# EUROPEAN PATENT APPLICATION

(11) **EP 1 269 998 A1**
(43) Date of publication of application: **02.01.2003**
(21) Application number: 01500248.8
(22) Date of filing: 18.10.2001
(51) Int. Cl.: A61K 9/58, A61K 31/18

(54) **Composition of prolonged liberation in the form of multiple granules that enables the maintenance of the therapeutic action of 4-nitro-2-phenoximethanesulfonanilide**

(30) Priority: 29.06.2001 AR 0103139
(71) Applicant: Bindecor, S.A., 1069 Buenos Aires (AR)
(72) Inventor: Gold, Oscar, 1069 Buenos Aires (AR)
(74) Representative: Urizar Anasagasti, José Antonio

(57) **Abstract**

Compound of prolonged action that includes two types of sugar-starch micro-granules which contain 4-nitro-2-phenoximethanesulfonanilide characterized in that:
the granules of the first type are of immediate or conventional liberation, in which the active principle, associated with a disintegrant, is incorporated using solutions of binding polymers.

The granules of the second type namely of controlled and prolonged action are prepared covering the micro-granules of the first type with solutions containing polymers that form a film in which it is possible to include plastifiers.

These two types of micro-granules are related in the final product in determined proportions, in order to achieve the desired profile of liberation.

## Description

### Field of the invention

The present invention refers to a compound of prolonged liberation in the form of multiple granules that enable the maintenance of the therapeutic action of 4-nitro-2-phenoximethanesulfonanilide, with a decrease in the oscillations of plasmatic concentrations. This active principle is sold under the trademark "NIMESULIDE".

Nimesulide is a non-steroid anti-inflammatory agent with anti-inflammatory, analgesic and anti-pyretic properties indicated for: osteoarthritis, rheumatoid arthritis, chronical inflammatory states of the superior respiratory tract, inflammation of the otolaryngologycal sphere, soft tissues, genital-urinary tract, dysmenorrhea, thrombophlebitis, phlebitis, odontalgia with adverse effects of the digestive type.

### Prior Art

Nimesulide or 4-nitro-2-phenoximethanesulfonanilide, is known since 1974 from the Belgian patent n. 804,812 and USA patent n. 3.840.897. Both patents define Nimesulide in its preparation and anti-inflammatory activity.
* USA N. 6.187.343, "Proceeding for the preparation of the prolonged action granule compound containing 4-nitro-2-phenoximethanesulfonanilide";
* Spanish N. 9.702.307 "Prolonged action compound in the form of granules containing 4-nitro-2-phenoximethanesulfonanilide".
* Italian N 1.297.032: "Proceeding for the preparation of the prolonged action granule compound containing 4-nitro-2-phenoximethanesulfonanilide";
define procedures for the preparation of formulations of the type claimed in said application.

The applicant, Bindecor S.A., Paraguay 1246, Montevideo, Uruguay, has acquired the rights of the abovementioned patents before the deposit of said application.

### Main Object

According to the specified ends, said invention refers to a compound of prolonged action that includes in its formulation two types of sugar-starch micro-granules, in which 4-nitro-2-phenoximethanesulfonanilide is included. Said granules allow the gradual liberation of said active principle, allowing the plasmatic concentrations therein to remain stable.

The granules of the first type are of immediate or conventional liberation, in which the active principle is incorporated using solutions of binding polymers.

The granules of the second type namely of controlled and prolonged action are prepared covering the micro-granules of the first type with solutions containing polymers that form a film.

These two types of micro-granules are related in the final product in determined proportions, in order to achieve the desired profile of liberation.

### Summary of the invention

In general terms, the present invention, refers to a medicine in the form of granules, its composition achieving an association of the active principle with the other components, allowing more stable plasmatic concentrations and reducing secondary effects of the digestive type.

In its composition, the medicine includes two types of sugar-starch micro-granules, in which the active principle is incorporated in association with a disintegrant.

The granules of the first type are of immediate or conventional liberation, in which the active principle is incorporated using solutions of binding polymers.

The granules of the second type namely of controlled and prolonged action are prepared covering the micro-granules of the first type with solutions containing polymers that form a film.

These two types of micro-granules are related in the final product in determined proportions, in order to achieve the desired profile of liberation.

The bonding polymers may be different types of polyvynilpyrrolidones, polyethylenglycols, jellies or their mixtures in acetonic, alcoholic, aqueous solution or mixtures thereof.

The cover polymers used may be different types of methylceluloses, different types of hydroxipropylmethylceluloses, phtalates of hydroxypropylmethylceluloses, acrylic polymers (Eudragit L, Eudragit S, Eudragit RL or Eudragit RS or their combinations), shellac and ethylceluloses; all these polymers may be combined in different proportions in acetonic, alcoholic, aqueous solution or mixtures thereof. To this solutions it is possible to incorporate plastifiers such as diethylphtalate, dibuthylphtalate, polyethylenglycol, triethylcitrate, triacetine, triglycerids of fatty acids, or others.

The proportion of the active principle respecting the rest of the excipients must be between 20% and 90%, and the one of bonding polymers with respect to the rest of the excipients between 0,2% and 5% and of disintegrants between 0,5% and 7%.

The solvent used in them different phases of elaboration may be 0% to 100% organic or 0% to 100% aqueous.

The proportion of granules with the covering for prolonged liberation with respect to the final mixture may be 0% to 50%.

The granules containing 4-nitro-2-phenoximethanesulfonanilide may be prepared such that the administration of different doses may be possible, due to this they may be dosed in capsules of hard jelly in different concentrations.

The association in granules works as a programmed and sustained dissolution system of the active principle. This profile of liberation can be controlled with the Dissolution Equipment USP-type XXIII, page 1791 of the basket type at 100 r.p.m. and each glass with 500ml of digestive solution, realizing the change in pH in the different hour fractions.

The profile of the solution is the following:

| | | |
|---|---|---|
| 1st hour | 20% | 50% |
| 4th hour | 55% | 85% |
| 8th hour | | > 80% |

With this invention, one can achieve ways of dosing of 4-nitro-2-phenoximethanesulfonanilide of prolonged and sustained action with stable hematic concentrations.

### Examples

The invention is further defined by means of the following examples:

### Example 1

| | |
|---|---|
| Neutrals | 120g |
| Sodium croscaramelose | 8g |
| Polyvynilpyrrolidine | 4g |
| Phtalate hydroxipropylmethylcelulose | 85g |
| Eudragit RL | 50g |
| Shellac | 95g |
| Triethylcitrate | 16g |
| Active principle | 615g |

1. In a double-cone mixer the active principle and the sodium croscaramelose are mixed; this association is micronized to a size inferior to 40 micras.
2. The resulting powder in 1 is incorporated slowly to a stainless steel pan that rotates at a speed between 8 and 30 r.p.m. in which the neutral granules have already been incorporated.
   The adding of the micronized material is simultaneous to the atomization of a solution over the neutrals; said solution is of polyvynilpyrrolidone in isopropylic alcohol. (4 g solute-36 g solvent)
   Once the process is finished the product is left to dry.
3. From the resulting product 35% of 2 is separated in weight and the 65% left is coated in a pan that works at a speed between 8 and 30 r.p.m., atomizing over the granules in order to coat them with a solution containing solutes (phtalate hydroxipropylmethylcelulose, Eudragit RL, shellac, triethylcitrate) (246g) dissolved in (1.400 g) of solvent with the proportion of 50% isopropylic alcohol, 40% acetone and 10% water.
4. The product resulting in 3 is dried, previously silicon dioxide is added and is mixed with the granules from 2.

### Example 2

| | |
|---|---|
| Neutrals | 120g |
| Crospovidone | 11g |
| Polyethylenglivcol | 3g |
| Phtalate hydroxipropylmethylcelulose | 45g |
| Hydroxipropylmethylcelulose | 52g |
| Eudragit RS | 27g |
| Ethylcelulose | 50g |
| Triacetine | 10g |
| Silicon dioxide | 4g |
| Active principle | 675g |

1. In a double-cone mixer the active principle and the crospovidone are mixed; this association is micronized to a size inferior to 40 micras.
2. The resulting powder in 1 is incorporated slowly to a stainless steel pan that rotates at a speed between 8 and 30 r.p.m. in which the neutral granules have already been incorporated.
   The adding of the micronized material is simultaneous to the atomization of a solution over the neutrals; said solution is of polyethylenglycol in water. (3g solute-10 g solvent)
   Once the process is finished the product is left to dry.
3. From the resulting product 35% of 2 is separated in weight and the 70% left is coated in a pan that works at a speed between 8 and 30 r.p.m., atomizing over the granules in order to coat them with a solution containing solutes (phtalate hydroxipropylmethylcelulose, Eudragit RS, ethylcelulose, triacetine) (184g) dissolved in (1.200 g) of solvent with the proportion of 70% isopropylic alcohol, 25% acetone and 50% water.
4. The product resulting in 3 is dried, previously silicon dioxide is added and is mixed with the granules from 2.

### Example 3

| | |
|---|---|
| Neutrals | 120g |
| Sodium croscaramelose | 4g |
| Crospovidone | 5g |
| Polyvynilpyrrilidone | 5g |
| Phtalate hydroxipropylmethylcelulose | 30g |
| Eudragit RL | 30g |
| Eudragit L | 29g |
| Shellac | 110g |
| Dibutylphtalate | 8g |
| Silicon dioxide | 2g |
| Active principle | 608g |

1. In a double-cone mixer the active principle, sodium croscaramelose and crospovidone are mixed; this association is micronized to a size inferior to 40 micras.
2. The resulting powder in 1 is incorporated slowly to a stainless steel pan that rotates at a speed between 8 and 30 r.p.m. in which the neutral granules have already been incorporated.
   The adding of the micronized material is simultaneous to the atomization of a solution over the neutrals; said solution is of polyvynilpyrrolidone in isopropylic alcohol. (5g solute-40 g solvent)
   Once the process is finished the product is left to dry.
3. From the resulting product 42% of 2 is separated in weight and the 58% left is coated in a pan that works at a speed between 8 and 30 r.p.m., atomizing over the granules in order to coat them with a solution containing solutes (phtalate hydroxipropylmethylcelulose, Eudragit RL, Eudragit L shellac, dibutylphtalate) (207g) dissolved in (1.500 g) of solvent with the proportion of 75% isopropylic alcohol, 20% acetone and 5% water.
4. The product resulting in 3 is dried, previously silicon dioxide is added and is mixed with the granules from 2.

### Example 4

| | |
|---|---|
| Neutrals | 120g |
| Sodium Croscaramelose | 10g |
| Polyvynilpyrrolidone | 3g |
| Eudragit RS | 25g |
| Eudragit L | 60g |
| Eudragit S | 10g |
| Shellac | 70g |
| Ethylcelulose | 15g |
| Triacetine | 3g |
| Triethylcitrate | 14g |
| Silicon dioxide | 3g |
| Active principle | 620g |

1. In a double-cone mixer the active principle and sodium croscaramelose are mixed; this association is micronized to a size inferior to 40 micras.
2. The resulting powder in 1 is incorporated slowly to a stainless steel pan that rotates at a speed between 8 and 30 r.p.m. in which the neutral granules have already been incorporated.
   The adding of the micronized material is simultaneous to the atomization of a solution over the neutrals; said solution is of polyvynilpyrrolidone in isopropylic alcohol and acetone. (5g solute-40 g solvent 95% isopropylic alcohol and 5% acetone)
   Once the process is finished the product is left to dry.
3. From the resulting product 33% of 2 is separated in weight and the 67% left is coated in a pan that works at a speed between 8 and 30 r.p.m., atomizing over the granules in order to coat them with a solution containing solutes (, Eudragit RS, Eudragit S, Eudragit L, shellac, ethylcelulose, triacetine, triethylcitrate) (197g) dissolved in (1.500 g) of solvent with the proportion of 50% isopropylic alcohol, 40% acetone and 10% water.
4. The product resulting in 3 is dried, previously silicon dioxide is added and is mixed with the granules from 2.

### Example 5

| | |
|---|---|
| Neutrals | 120g |
| Sodium croscaramelose | 10g |
| Polyvynilpyrrolidone | 4g |
| Phtalate hydroxipropylmethylcelulose | 95g |
| Eudragit RL | 60g |
| Shellac | 87g |
| Triethylcitrate | 14g |
| Silicon dioxide | 5g |
| Active principle | 608g |

1. In a double-cone mixer the active principle and sodium croscaramelose are mixed; this association is micronized to a size inferior to 40 micras.
2. The resulting powder in 1 is incorporated slowly to a stainless steel pan that rotates at a speed between 8 and 30 r.p.m. in which the neutral granules have already been incorporated.
   The adding of the micronized material is simultaneous to the atomization of a solution over the neutrals; said solution is of polyvynilpyrrolidine in isopropylic alcohol. (4g solute-14 g solvent)
   Once the process is finished the product is left to dry.
3. From the resulting product 35% of 2 is separated in weight and the 70% left is coated in a pan that works at a speed between 8 and 30 r.p.m., atomizing over the granules in order to coat them with a solution containing solutes (phtalate hydroxipropylmethylcelulose, Eudragit RL, shellac, triethylcitrate) (256g) dissolved in (2.300 g) of solvent with the proportion of 85% isopropylic alcohol, 11% acetone and 4% water.
4. The product resulting in 3 is dried, previously silicon dioxide is added and is mixed with the granules from 2.

The Test of Liberation of the granules was effected according to U.S.P. XXIII, basket method with 500ml glass changing the pH in hourly fractions obtaining the following results:

| Liberation Percentage | | | | | |
|---|---|---|---|---|---|
| Hs. | Example 1 | Example 2 | Example 3 | Example 4 | Example 5 |
| 1 | 35 | 31 | 43 | 34-40 | |
| 4 | 76 | 72 | 80 | 70-77 | |
| 8 | 95 | 91 | 99 | 91-98 | |

### Pharmacocinetical studies

Bearing in mind the abovementioned and the example presented, capsules with granules or microgranules of 200 mg of 4-nitro-2-phenoximethanesulfonanilide of controlled action were produced, and comparative pharmacocinetic studies were with the sold products of conventional action with a dosage of 100mg and 200mg.

as significant parameters the maximum plasmatic concentration (Cmax), the time to reach the maximum concentration (Tmax) and the area under the curve (AUC) was calculated in each one of the products. The studies were done with 6 individuals for each one of the 3 products which were administered with a dose orally and extractions were effected at different hours, analyzing the plasmatic concentrations, and realizing the statistical study of the calculated parameters.

All along the evaluation, the products are defined in the following way:
LP3 microgranules of prolonged or controlled action of 4-nitro-2-phenoximethanesulfonanilide in hard jelly capsules with a 200mg dose.
100mg 100mg dose of Nimesulide in tablets of conventional action.
200mg 200mg dose of Nimesulide in tablets of conventional action.

The characteristics to be considered in relation to the prolonged action product with respect to the 100mg and 200mg conventional products are the following:
the LP3 product has plasmatic levels compared with the 100mg product which are similar during the first three hours but are more stable and superior in the following hours. With respect to the 200mg. product it has plasmatic concentrations which are more stable and superior during the first 5-6 hours maintaining similar levels in the following hours.

The LP3 product with these characteristics is defined as of controlled action and it is demonstrated in Table I and Figures I and II enclosed herein, observing a significant shift of Tmax, and Cmax and a AUC with no significant differences respecting the 200mg product and at the same time with concentrations which are stable through the hours.

Table I and Figures II and II show the parameters of granules of 4-nitro-2-phenoximethanesulfonanilide, conventional Nimesulide 100mg. and conventional Nimesulide 200mg. (average values of 6 individuals). In the enclosed graphs:
Figure 1 shows the average concentrations along the time of granules 4-nitro-2-phenoximethanesulfonanilide and Nimesulide 100mg (average values of 6 individuals) while,
figure 2 shows average concentrations along the time of granules of 4-nitro-2-phenoximethanesulfonanilide, conventional Nimesulide 100mg and conventional Nimesulide 200mg(average value of 6 individuals).

**TABLE I**

| Formulation | Cmax Ug/ml | Tmax (hours) | AUC(ug.h/ml) (ug.h/ml) □ | F (LP3/conventional dose) |
|---|---|---|---|---|
| LP3 | 4,6 +- 0,328 | 3,7+-0,211 | 37,1 +- 3,17 | ---------------- |
| 100mg | 3,8 +- 0,122 | 1,5+-0,342 | 20,0 +- 1,73 | 0,93+- 0,063 |
| 200mg | 7,5 +- 0,389 | 1,8+-0,543 | 42,6+- 5,06 | 0,90+- 0,066 |

The present invention has been thus described as an example in order to allow the skilled in the art to understand and carry out the fundaments of the invention, but it is indubitable that modifications can be produced without parting form the spirit and scope of the invention.

## Claims

1. Compound of prolonged action that includes two types of sugar-starch micro-granules which contain 4-nitro-2-phenoximethanesulfonanilide **characterized in that**:
the granules of the first type are of immediate or conventional liberation, in which the active principle, associated with a disintegrant, is incorporated using solutions of binding polymers.
The granules of the second type namely of controlled and prolonged action are prepared covering the micro-granules of the first type with solutions containing polymers that form a film in which it is possible to include plastifiers.
These two types of micro-granules are related in the final product in determined proportions, in order to achieve the desired profile of liberation.

2. Compound according to claim 1 wherein the neutral nucleus have a size of 0,2 mm to 1,8 mm, preferably 0,4 mm to 1,4mm.

3. Compound according to claim 1 wherein the active principle is mixed or associated to disintegrants such as sodium croscaramelose, crospovidone, alone or combined, before being incorporated to the micro-granules.

4. Compound according to claim 1 wherein:
(A) the bonding polymers that adhere to the active principle are selected from the group of polyvynilpyrrolidones and polyethylenglycols, jellies alone or combined; and
(B) the polymers of the fraction coating the active principle and that confer prolonged and controlled action to the active principle are selected form the group of different types of hydroxipropylmethylcelulose, phtalate hydroxipropylmethylcelulose, ethylceluloses, shellac, different types of metacrylates (Eudragit L, Eudragit S, Eudragit RL, Eudragit RS) or their combinations.

5. Compound according to claim 1 wherein the percentage of granules of immediate action in the mixture or association with the granules of prolonged and controlled action may 0% to 50%.

6. Proceeding according to claim 1 wherein in (iii) the solution contains plastifiers selected from a group including diethylphtalate, polyethylenglycol, dibuthylphtalate, triacetine, triethylcitrate, triglycerids of fatty acids alone or combined.

7. Compound according to claim 1 wherein the proportion of active principle respecting the rest of the excipients must be between 20% and 90%.

8. Compound according to claim 1 wherein the proportion of bonding polymers with respect to the rest of the excipients has to be included between 0,2% and 5%.

9. Compound according to claim 1 wherein the proportion of disintegrants must be between 0,5% and 7%.

10. Compound according to claim 1 wherein the percentage of metacrylates in proportion with the rest of the polymers is between 0% and 100%,

11. Compound according to claim 1 wherein the final size of the granules is 0,6mm to 2 mm, preferably between 0,8mm and 1,7mm.

12. Compound according to claim 1 wherein the granules maintain stable plasmatic concentrations and similar to the dose of less concentration during 3 hours and without no significant differences with the one of most concentration of active principle in the following hours.
